# EUROPEAN PATENT APPLICATION

(11) **EP 4 053 311 A1**
(43) Date of publication of application: **07.09.2022**
(21) Application number: 20883596.7
(22) Date of filing: 26.10.2020
(51) Int. Cl.: C40B 40/06, C40B 40/10, C07K 19/00

(54) **HALF-LIFE EXTENSION DRUG AND LIBRARY THEREOF, AND PREPARATION METHOD AND APPLICATION THEREOF**

(30) Priority: 29.10.2019 CN 201911038880
(71) Applicant: Assembly Medicine, LLC, Shanghai 201203 (CN)
(72) Inventor: CHOU, James Jeiwen, Shanghai 201203 (CN); PAN, Liqiang, Shanghai 201203 (CN); RUN, Changqing, Shanghai 201203 (CN); ZHOU, Liujuan, Shanghai 201203 (CN)
(74) Representative: Lavoix
(86) International application number: PCT/CN2020/123685
(87) International publication number: WO 2021/083077

(57) **Abstract**

The present invention provides a half-life extension drug and a library thereof, and a preparation method and application thereof. Specifically, the present invention provides a drug library, comprising: (a) a drug unit; and (b) n half-life extension units, wherein the drug unit comprises a drug element portion and a first nucleic acid element portion connected to the drug element portion, and each half-life extension unit comprises a half-life extension element portion and a second nucleic acid element portion connected to the half-life extension element portion. Moreover, one first nucleic acid element portion of the drug unit and the second nucleic acid element portion of the at least one half-life extension unit may form a "drug unit-half-life extension unit" complex by forming a complementary base pairing structure, and n is a positive integer greater than or equal to 1.

sequence listing

<110>  ASSEMBLY MEDICINE, LLC.

<120>  HALF-LIFE EXTENSION DRUG AND LIBRARY THEREOF, AND PREPARATION METHOD
AND APPLICATION THEREOF

<130>  P2020-2077

<150>  CN201911038880.0
<151>  2019-10-29

<160>  8

<170>  PatentIn version 3.5

<210>  1
<211>  29
<212>  DNA
<213>  artificial sequence

<220>
<223>  L-DNA1

<400>  1
aagaggacgc aatcctgagc acgaggtct                                          29


<210>  2
<211>  29
<212>  DNA
<213>  artificial sequence

<220>
<223>  L-DNA2

<400>  2
aactgctgcc atagtggatt gcgtcctct                                          29


<210>  3
<211>  29
<212>  DNA
<213>  artificial sequence

<220>
<223>  L-DNA3

<400>  3
aatgagtgca ttcggactat ggcagcagt                                          29


<210>  4
<211>  29
<212>  DNA
<213>  artificial sequence

<220>
<223>  L-DNA4

<400>  4
aagacctcgt gctcaccgaa tgcactcat                                          29

<210>  5
<211>  155
<212>  PRT
<213>  artificial sequence

<220>
<223>  Anti-HSA Nanobody

<400>  5

Ser Ala Trp Ser His Pro Gln Phe Glu Lys Gly Gly Gly Ser Gly Gly
1               5                   10                  15

Gly Ser Gly Gly Ser Ala Trp Ser His Pro Gln Phe Glu Lys Glu Asn
            20                  25                  30

Leu Tyr Phe Gln Ser Glu Val Gln Leu Val Glu Ser Gly Gly Gly Leu
        35                  40                  45

Val Gln Pro Gly Asn Ser Leu Arg Leu Ser Cys Ala Ala Ser Gly Phe
    50                  55                  60

Thr Phe Ser Ser Phe Gly Met Ser Trp Val Arg Gln Ala Pro Gly Lys
65                  70                  75                  80

Gly Leu Glu Trp Val Ser Ser Ile Ser Gly Ser Gly Ser Asp Thr Leu
                85                  90                  95

Tyr Ala Asp Ser Val Lys Gly Arg Phe Thr Ile Ser Arg Asp Asn Ala
            100                 105                 110

Lys Thr Thr Leu Tyr Leu Gln Met Asn Ser Leu Arg Pro Glu Asp Thr
        115                 120                 125

Ala Val Tyr Tyr Cys Thr Ile Gly Gly Ser Leu Ser Arg Ser Ser Gln
    130                 135                 140

Gly Thr Leu Val Thr Val Ser Ser Gly Ser Cys
145                 150                 155


<210>  6
<211>  18
<212>  PRT
<213>  artificial sequence

<220>
<223>  SA21

<400>  6

Arg Leu Ile Glu Asp Ile Cys Leu Pro Arg Trp Gly Cys Leu Trp Glu
1               5                   10                  15

Asp Asp

<210> 7
<211> 11
<212> PRT
<213> artificial sequence

<220>
<223> core sequence

<400> 7

Asp Ile Cys Leu Pro Arg Trp Gly Cys Leu Trp
1               5                   10

<210> 8
<211> 30
<212> RNA
<213> artificial sequence

<220>
<223> RNA-type nucleic acid aptamer

<400> 8
guggacuaua ccgcguaaug cugccuccac 30

## Description

### Technical field

The present invention relates to the field of biotechnology medicines. In particular, it relates to drugs with prolonged half-life and libraries thereof, as well as preparation methods and uses.

### Background

For protein-based drugs, prolonging their half-life is beneficial for improving efficacy and reducing drug dosage.

In order to prolong the half-life of protein or polypeptide drugs, existing methods include preparing them in the form of: Fc-fusion protein, HSA-fusion protein; using chemical coupling to prepare Fc-protein/polypeptide conjugate, HSA-protein /polypeptide conjugate; and replacing HSA in the above fusion proteins or conjugates with proteins or polypeptides (such as anti-HSA antibodies), small molecules, nucleic acids (such as aptamer), etc. that can bind to HSA; PEG, glycosylation , sialic acid and other chemical modification methods.

PEG modification is the main chemical modification method to improve the half-life of protein or polypeptide drugs. The advantage is that PEG has strong biocompatibility and can increase the solubility and stability of drugs. The disadvantage is: PEG modification will greatly affect the activity of active proteins such as biological enzymes, large molecular weight PEG has immunogenicity, and poor PEG molecular homogeneity makes it difficult to control the quality of PEGylated drugs. In addition, the half-life improvement of protein or polypeptide drugs by chemical modification methods such as PEG depends on the increase of molecular weight, so that the half-life improvement effect is limited.

Although Fc or HSA fusion protein can prolong the half-life, the method of prolonging the half-life of Fc or HSA fusion protein will generate new epitopes at the fusion site of the target protein or polypeptide and Fc/HSA. In addition, Fc can only be fused to the C-terminus, while HSA can only be fused to the N-terminus or C-terminus of the target protein or polypeptide. Furthermore, this method is not applicable to native proteins or peptides.

Therefore, there is an urgent need in the art to develop a simple, flexible, efficient and modular connection method that can specifically heterologously couple protein drugs with half-life extension factors (referring to HSA, HSA binding modules, etc.), thereby prolonging the half-life of protein drugs, improving their pharmacokinetic properties.

### Summary of the invention

The purpose of the present invention is to provide a simple, flexible, efficient and modular method for the specific heterologous conjugation of protein drugs and half-life prolonging factors and the conjugated drugs prepared therefrom.

Another object of the present invention is to provide a low-cost, modularized FcRn targeting unit and a module library composed of the modularized FcRn targeting unit. The FcRn targeting unit can be used as a half-life improving module, flexibly linked with protein drugs, thereby significantly prolonging the half-life of protein drugs.

In a first aspect of the present invention, it provides a drug library comprising: (a) a drug unit; and (b) n half-life extension units;
wherein, the drug unit comprises a drug component part and a first nucleic acid component part connected to the drug component part;
the half-life extension unit comprises a half-life extension component part and a second nucleic acid component part connected to the half-life extension component part;
and one of the first nucleic acid component part of the drug unit and the second nucleic acid component part of at least one half-life extension unit can form a base-complementary pairing structure to form a "drug unit-half-life extension unit" complex;
wherein, n is a positive integer ≥ 1.

In another preferred embodiment, the drug unit and the half-life extension unit can be directly or indirectly connected by base complementary.

In another preferred embodiment, the " base complementary" includes direct base complementary and/or indirect base complementary.

In another preferred embodiment, the "direct base complementary" includes the direct formation of base complementary between the first nucleic acid component part and the second nucleic acid component part.

In another preferred embodiment, the "indirect base complementary" includes the first nucleic acid component part and the second nucleic acid component part, through the single-stranded complementary sequences of one or more (such as 2, 3, 4, 5, 6) auxiliary complementary nucleic acid molecules (i.e., nucleic acid F) undergoing base complementary, thereby forming a triple or more pairing structure.

In another preferred embodiment, the auxiliary complementary nucleic acid molecule is in single-stranded form.

In another preferred embodiment, the auxiliary complementary nucleic acid molecule is selected from the group consisting of an independent nucleic acid molecule, a second nucleic acid component part of another half-life extension unit, a first nucleic acid component part of another drug unit, and a combination thereof .

In another preferred embodiment, the auxiliary complementary nucleic acid molecule is a nucleic acid that is not coupled to a protein drug.

In another preferred embodiment, the auxiliary complementary nucleic acid molecule is a first nucleic acid component part of another different drug unit, and/or a second nucleic acid component part of another different half-life extension unit.

In another preferred embodiment, the first nucleic acid component part, the second nucleic acid component part, and/or the auxiliary complementary nucleic acid molecule is L-nucleic acid, or a nucleic acid modified by a modifying group.

In another preferred embodiment, the first nucleic acid component is not connected to the N-terminus (amino terminal) of the drug component part, and/or which is also not connected to the C-terminus (carboxy-terminal) of the drug component part.

In another preferred embodiment, the first nucleic acid component is linked to a non-terminal amino acid residue portion of the drug component part.

In another preferred embodiment, the second nucleic acid component is not connected to the N-terminus (amino terminal) of the half-life extension component part, and/or which is not connected to the C-terminus (carboxy-terminal) of the half-life extension component part.

In another preferred embodiment, the second nucleic acid component is linked to a non-terminal amino acid residue portion of the half-life extension component part.

In another preferred embodiment, the half-life extension component part in the half-life extension unit is not Fc or a fragment thereof or modified Fc or a fragment thereof (e.g., glycosylated Fc, NH₂ modified Fc).

In another preferred embodiment, the half-life extension component part in the half-life extension unit is not PEG (polyethylene glycol).

In another preferred embodiment, the "drug unit-half-life extension unit" complex has one half-life extension unit.

In another preferred embodiment, the "drug unit-half-life extension unit" complex has two or more different half-life extension units.

In another preferred embodiment, in the "drug unit-half-life extension unit" complex, the molar ratio (A/D) of the half-life extension unit to the drug unit is 0.5-10, preferably 1-8, more preferably 2-5.

In another preferred embodiment, the "drug unit-half-life extension unit" complex has one drug unit.

In another preferred embodiment, the "drug unit-half-life extension unit" complex has two or more different drug units.

In another preferred embodiment, in the "drug unit-half-life extension unit" complex, the molar ratio (A/D) of the half-life extension unit to the drug unit is 0.5-10, preferably 1-8, more preferably 2-5.

In another preferred embodiment, the drug component part is a protein drug.

In another preferred embodiment, the drug component part is a polypeptide drug.

In another preferred embodiment, the drug component part is a fusion protein drug.

In another preferred embodiment, the "drug unit-half-life extension unit" complex has a structure as shown in Formula I:

To-[Lo-Ao]n (I)

wherein
T₀ is a drug component part;
L₀ is a nucleic acid linker (linker), the nucleic acid linker is used to connect T₀ and A₀ and contains a pairing structure based on the base complementary of the first nucleic acid component part and the second nucleic acid component part;
A₀ is an FcRn binding component, or a binding component that binds to an FcRn binding protein such as serum albumin;
n is a positive integer ≥1.

In another preferred embodiment, the drug unit has a structure shown in Formula II:

T-X1-L1-Y1-W1-Z1 (II)

the half-life extension unit has a structure shown in Formula III:

A-X2-L2-Y2-W2-Z2 (III)

wherein
T is a drug component part;
A is a half-life extension component part;
X1 and X2 is each independently none or a redundant peptide;
L1 and L2 is each independently a linker molecule;
Y1, Y2 and Z1, Z2 is each independently none or a redundant nucleic acid;
W1 and W2 is each independently a nucleic acid sequence selected from the group consisting of L-nucleic acid, peptide nucleic acid, locked nucleic acid, thio-modified nucleic acid, a 2'-fluoro-modified nucleic acid, a 5-hydroxymethylcytosine nucleic acid, and a combination thereof;
"-" is a covalent bond;
wherein the nucleic acid W1 of the drug unit has at least one complementary pairing region, and the nucleic acid W2 of the half-life extension unit has at least one complementary pairing region, and the two are partially or completely complementary.

In another preferred embodiment, the drug component part is selected from the group consisting of an antibody, an activation receptor or inhibition receptor, a ligand of the protein, a biologically active enzyme, a nucleic acid drug, a small molecule drug, and a combination thereof.

In another preferred embodiment, the half-life extension component part is selected from the group consisting of: a natural albumin (Albumin), recombinant albumin, anti-albumin antibody (including nanobody, single chain antibody, Fab, monoclonal antibody), a nucleic acid aptamer (aptamer) that specifically bind to albumin, a protein and a nucleic acid aptamer (aptamer) that binds directly to FcRn, any protein with a long half-life, and a combination thereof.

In another preferred embodiment, X1 and X2 is each independently 0-30 amino acids.

In another preferred embodiment, X1 and X2 is independently 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 amino acids.

In another preferred embodiment, the linker molecule L1 and L2 each independently has a bifunctional linker, which can be coupled with the modified ends with modifying groups of nucleic acids W1, W2 or Y1, Y2 and T or A or the specific linking site of XI, X2.

In another preferred embodiment, the active groups of the linker molecules L1 and L2 are each independently selected from: maleimide, haloacetyl, and thiopyridine.

In another preferred embodiment, the haloacetyl group is selected from: iodoacetyl and bromoacetyl.

In another preferred embodiment, the drug component part T is a protein drug component.

In another preferred embodiment, the protein drug component T is a wild type or a mutant type.

In another preferred embodiment, the mutation does not affect the drug function.

In another preferred embodiment, the mutation comprises the introduction of one or more cysteine residues (Cys) into the protein drug component.

In another preferred embodiment, the cysteine residue is located at any position of the protein drug component (e.g., N-terminal, C-terminal, or any position in the middle).

In another preferred embodiment, the mutation comprises the introduction of one or more cysteine residues at the carboxy terminus (C-terminus) of a protein drug component (e.g., an antibody).

In another preferred embodiment, the cysteine residues are used to connect DNA.

In another preferred embodiment, the protein drug component is FVIII.

In another preferred embodiment, Y1 and Y2 are each independently 0-30 nucleotides.

In another preferred embodiment, each of the Y1 and Y2 is independently a L-nucleic acid.

In another preferred embodiment, Y1 and Y2 are independently 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides.

In another preferred embodiment, the Y1 and Y2 are each independently AAAA, AAA or AA.

In another preferred embodiment, Z1 and Z2 are each independently 0-30 nucleotides.

In another preferred embodiment, each of the Z1 and Z2 is independently L-nucleic acid.

In another preferred embodiment, Z1 and Z2 are independently 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 nucleotides.

In another preferred embodiment, the Z1 and Z2 are each independently AAAA, AAA or AA.

In another preferred embodiment, the nucleic acids W1 and W2 are L-nucleic acid.

In another preferred embodiment, the nucleic acids W1 and W2 are selected from the group consisting of DNA and RNA.

In another preferred embodiment, the modifying group is selected from the group consisting of NH₂, alkynyl, sulfydryl (SH), carboxyl (COOH), and a combination thereof.

In another preferred embodiment, the modifying group is NH₂.

In another preferred embodiment, the position of the modifying group on the nucleic acid W1, W2 and/or Y1, Y2 is selected from: 5' end, 3' end, and any position in the middle.

In another preferred embodiment, a transition region with a length of 0-10 nt is located between any two complementary pairing regions in the nucleic acids W1 and W2.

In another preferred embodiment, the transition region is AAAA, AAA or AA.

In another preferred embodiment, the length of the complementary pairing region is 5-100nt; preferably 8-50nt; more preferably 10-30nt; more preferably 12-25nt; most preferably 16-20nt .

In a second aspect of the present invention, it provides a method assembling a drug with extended half-life, such as a protein drug, comprising:
(a) selecting a drug unit and a half-life extension unit from the drug library according to the first aspect of the present invention based on pharmaceutical information; and
(b) mixing the drug unit and at least one half-life extension unit to assemble a drug with extended half-life.

In another preferred embodiment, the assembly is to form a base-complementary pairing structure (e.g., a double-stranded pairing structure) through the complementation of the nucleic acid component part.

In another preferred embodiment, in the drug with extended half-life, the nucleic acid component part of each drug unit forms a base-complementary pairing structure (for example, a double-stranded pairing structure) with one or two or three nucleic acid component parts of the half-life extension unit.

In another preferred embodiment, the assembly is to form a base-complementary pairing structure (e.g., a double-stranded pairing structure) by the complementation of the nucleic acid component part with the single-stranded complementary sequence of the auxiliary complementary nucleic acid molecule (i.e., nucleic acid F).

In another preferred embodiment, the auxiliary complementary nucleic acid molecule is in single-stranded form.

In another preferred embodiment, the nucleic acid F is a nucleic acid uncoupled to a protein drug.

In another preferred example, the nucleic acid F is a L-nucleic acid, or a nucleic acid modified with a modifying group.

In another preferred embodiment, the length of the nucleic acid F is 1-1.5 times the sum of the number of pairs of monomeric nucleic acids in all (b).

In another preferred embodiment, the pharmaceutical information is protein drug information required for treating the disease of the object to be treated, including the type, administration mode, and pharmacokinetics of the protein drug.

In another preferred embodiment, the assembly condition is: 5-50 degrees (preferably 25-40 degrees), and the reaction is performed for 1-15 minutes (preferably 5-10 minutes).

In another preferred embodiment, the assembly condition is: pH 6-8.

In a third aspect of the present invention, it provides a drug with extended half-life, the drug with extended half-life is a drug with extended half-life formed by a drug unit and n kinds of half-life extension units through nucleic acid complementation to form a base-complementary pairing structure (for example, a double-stranded pairing structure), wherein n is a positive integer ≥ 1;
wherein, the drug unit comprises a drug component part and a first nucleic acid component part connected to the drug component part, and the half-life extension unit comprises a half-life extension component part and a second nucleic acid component part connected to the half-life extension component part, and the nucleic acid component part of one of the drug units and the nucleic acid component part of at least one half-life extension unit form base-complementary pairing structures (e.g., double-stranded pairing structures) by complementation.

In another preferred embodiment, the nucleic acid component part is resistant to nuclease degradation.

In another preferred embodiment, the drug is a protein drug, and the protein drug has a structure as shown in Formula I:

To-[Lo-Ao]n (I)

wherein
T₀ is a protein drug;
L₀ is a nucleic acid linking element for connecting T₀ and A₀;
Ao is an FcRn binding component; or a binding component that binds to an FcRn binding protein such as serum albumin;
n is a positive integer >1.

In another preferred embodiment, the nucleic acid component part is selected from the group consisting of L-nucleic acid, peptide nucleic acid, locked nucleic acid, thio-modified nucleic acid, a 2'-fluoro-modified nucleic acid, a 5-hydroxymethylcytosine nucleic acid, and a combination thereof.

In another preferred embodiment, the drug unit and the half-life extension unit are the drug unit and the half-life extension unit from the drug library according to the first aspect of the present invention.

In another preferred embodiment, the drug with extended half-life is a multimeric protein drug, and the multimeric protein drug is a multimer formed by D kinds of protein drug monomers through nucleic acid complementation to form a base-complementary pairing structure (for example, a double-stranded pairing structure), wherein D is a positive integer ≥ 2.

In another preferred embodiment, D is a positive integer of 2-6; preferably D is 2, 3, 4 or 5.

In another preferred embodiment, the drug with extended half-life is a protein drug with extended half-life.

In another preferred embodiment, the degraded half-life HI of the protein drug with extended half-life in vivo is greater than the in vivo half-life H2 of the single protein drug component.

In another preferred embodiment, the ratio of H1/H2 is 1-100, preferably 10-50, more preferably 10-20.

In a fourth aspect of the present invention, it provides a pharmaceutical composition, the pharmaceutical composition comprises
(i) the drug with extended half-life according to the third aspect of the present invention is used as an active ingredient; and
(ii) a pharmaceutically acceptable carrier.

In another preferred embodiment, the dosage form of the pharmaceutical composition is selected from the group consisting of: injection, lyophilized agent.

It should be understood that, within the scope of the present invention, the technical features specifically described above and below (such as in the Examples) can be combined with each other, thereby constituting a new or preferred technical solution which needs not be described one by one.

### DESCRIPTION OF THE DRAWINGS

Figure 1 shows the mechanism diagram of prolonging the half-life of drugs by nucleic acid pairing in the present invention.
Figure 2 shows the structure of the protein drug linked to the FcRn targeting unit in an example of the present invention.
Figure 3 shows agarose gel electrophoresis images before and after four different L-DNA single strands form a tetrameric framework.
Figure 4 shows the half-life of long-acting coagulation factor VIII in a mouse model of hemophilia.
Figure 5 shows HSAnb protein purification and HSAnb-DNA ligation SDS-PAGE gel electrophoresis.

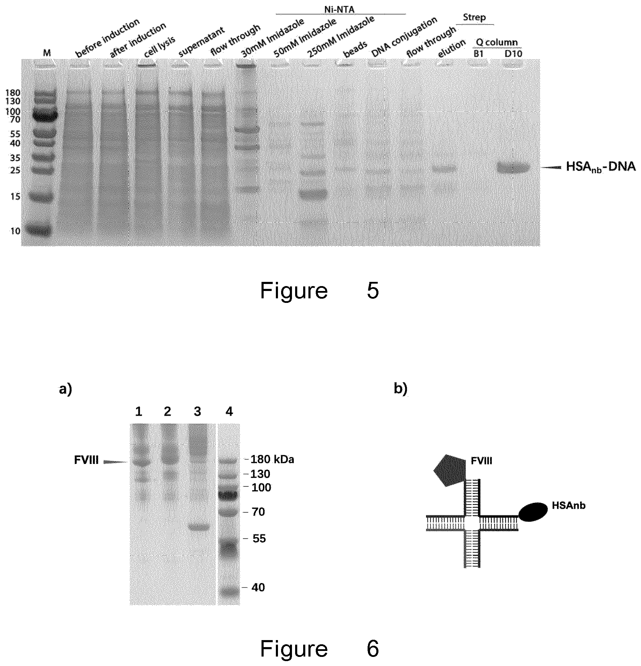

Figure 6 shows the SDS-PAGE electrophoresis gel image of the long-acting FVIII assembly results. Among them, (a) SDS-PAGE electrophoresis gel image of long-acting FVIII assembly results. Lane 1: FVIII; after FVIII powder is dissolved in ddH₂O, the buffer is replaced with PBS pH7.4 by desalting, the final concentration is 133 IU/ml, and 10ul is taken for SDS-PAGE loading; Lane 2: FVIII-L-DNA ligation results; FVIII with a final concentration of 133 IU/ml is ligated with L-DNA1-(PEG)2-MAL in a ratio of 1:4 (reaction at 25 degrees for 2h) to obtain a FVIII-DNA1 sample. Taking 10ul for SDS-PAGE loading; lane 3: FVIII-HSAnb assembly result; adding HSAnb-DNA4, L-DNA2 and L-DNA3 to the FVIII-DNA1 sample in turn, and mixed well. Ensuring that the molar ratio of L-DNA1, L-DNA2, L-DNA3 and L-DNA4 in the reaction system is 1:1:1:1, and assemble to obtain long-acting FVIII. Taking 10ul for SDS-PAGE loading; Lane 4: Protein molecular weight standard; b) Schematic diagram of the assembled structure of FVIII-HSAnb.
Figure 7 shows the in vitro activity APTT test results of samples FVIII, FVIII-L-DNA1, FVIII-HSAnb.

### DETAILED DESCRIPTION OF THE INVENTION

After extensive and in-depth research, the present inventors have developed a drug with extended half-life and its library, as well as its preparation method and application for the first time. Using the drug library and preparation method of the present invention, drugs with extended half-life can be assembled rapidly, efficiently, at low cost and in high yield as required. The present invention has been completed on this basis.

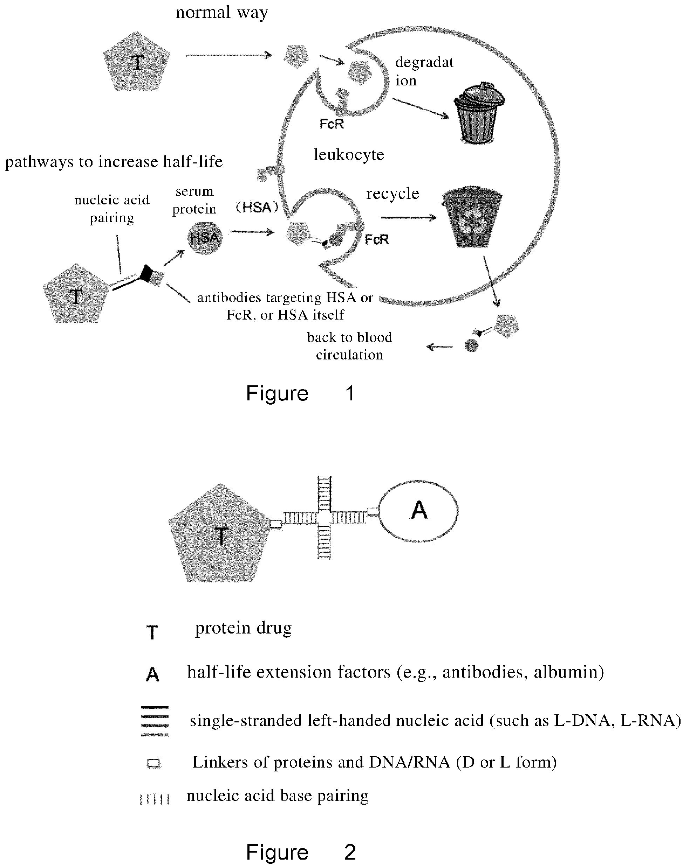

Specifically, the present invention provides a drug with extended half-life, which comprises: (a) a drug unit; and (b) n half-life extension units coupled to the drug unit through nucleic acid base complementation; wherein, the drug unit comprises a drug component part (moiety) and a first nucleic acid component part connected to the drug component part, and the half-life extension unit includes a half-life extension component part and a second nucleic acid component part connected to the half-life extension component part, and the nucleic acid component part of one of the drug units and the nucleic acid component part of at least one half-life extension unit can form a base-complementary pairing structure (such as a double-stranded pairing structure) through complementation, thereby constituting the drug; Among them, n is a positive integer ≥ 1, the drug with extended half-life of the present invention only forms a stable nucleic acid base complementary pairing structure (rather than complex peptide bonds or other chemical modifications, etc.) through rapid assembly (e.g., 1 minute). Experiments show that the drug of the present invention can specifically bind to FcRn through the FcRn binding component, or the binding component that binds to an FcRn binding protein (such as serum albumin). Unexpectedly, when the drug of the present invention is endocytosed by hematopoietic cells or vascular endothelial cells in the blood through pinocytosis, it can enter the endosome and bind to the FcRn therein, thereby entering the recycling pathway, and resistant to degradation in a variety of environments, including intracellular and endosome. In this way, the drug of the present invention can effectively avoid entering into the degradation pathway to be degraded by lysosome. In addition, after the drug of the present invention is circulated to the cell surface, it can be dissociated from FcRn under neutral physiological conditions (e.g., about pH 7.4) and released into the blood, thereby realizing recycling (see Figure 1).

### Terms

Unless otherwise defined, all technical and scientific terms used herein have the same meaning as commonly understood by one of ordinary skill in the art to which this invention belongs. As used herein, when used in reference to a specifically recited value, the term "about" means that the value may vary by no more than 1% from the recited value. For example, as used herein, the expression "about 100" includes all values between 99 and 101 and (e.g., 99.1, 99.2, 99.3, 99.4, etc.).

Although any methods and materials similar or equivalent to those described in the present invention can be used in the practice or testing of the present invention, the preferred methods and materials are exemplified herein.

As used herein, the terms "protein drug" and "polypeptide drug" are used interchangeably and refer to a drug containing amino acids through peptide bonds to form a polypeptide sequence, which may consist only of amino acid sequences or may consist essentially of amino acid sequences. In addition, the term also includes unmodified or modified forms, e.g., glycosylated protein drugs, PEGylated protein drugs, ADC forms of protein drugs.

As used herein, the terms "half-life extension unit," "half-life extension module," and "half-life enhancement module" are used interchangeably to refer to a component or module that contains a half-life extension component part and a second nucleic acid component part linked to said half-life extension component part and used to form a "drug unit-half-life extension unit" complex through base complementation, thereby extending the half-life of the protein-drug complex.

As used herein, the terms "protein drug complex" or "drug unit-half-life extension unit complex" are used interchangeably to refer to a drug complex formed by base complementation of a drug unit and a half-life extension unit.

### Drug T

In the present invention, the drug unit includes a drug component part and a first nucleic acid component part connected to the drug component part.

Wherein, the drug component part is a protein drug or a polypeptide drug.

Typically, the protein drugs include, but are not limited to, antibodies, fusion proteins, growth factors, hormones (e.g., insulin, growth hormone, etc.).

In a preferred embodiment of the present invention, the protein drug is a long-acting factor eight (FVIII) for the treatment of hemophilia A.

### Half-life extension unit A

In the present invention, the half-life extension unit includes a half-life extension component part and a second nucleic acid component part connected to the half-life extension component part.

Specifically, the present invention utilizes nucleic acid pairing-mediated multispecific antibody self-assembly technology (Patent Application No. 201710322583.3) to connect FcRn-targeting proteins or polypeptides (half-life extension factors) and drugs to nucleic acid elements respectively to form FcRn targeting units, which are then coupled together by complementary pairing of nucleic acid elements to form long-acting drugs that can be recovered by cells.

The half-life extension component part (half-life extension factor) can be divided into the following major categories:

### 2.1 HSA protein

The HSA protein can be HSA extracted from blood, or recombinantly expressed by low-cost expression systems such as yeast. HSA has a free Cys34 that can be used for site-directed coupling of activated single-stranded degradation-resistant nucleic acids (such as reverse nucleic acids). Of course, if site-specific coupling is not required, NH₂ on the surface of HSA can also be used for random coupling of single-stranded nucleic acids.

### 2.2 HSA-binding proteins

Types of HSA-binding proteins include nanobodies, single-chain antibodies, Fab, and full-length IgG antibodies. According to the present invention, the preferred HSA-binding proteins are nanobodies and single-chain antibodies with lower production costs.

### 2.3 HSA-binding polypeptides and domains

Polypeptides of a specific sequence can also bind to HSA, such as polypeptide SA21 (amino acid sequence: Ac-RLIEDICLPRWGCLWEDD-NH₂, SEQ ID NO. 6), wherein the core sequence is DICLPRWGCLW (SEQ ID NO. 7), and the polypeptides containing the core sequence can combine with HSA to varying degrees and become the half-life enhancing factor of the patent of the present invention.

Other protein domains can also become HSA-binding protein domains through directional modification, evolution and other means, such as artificially engineered small fragment antibodies such as Affibody and DART.

### 2.4 HSA-binding nucleic acids

Some aptamers can specifically bind to HSA, for example, the sequence of one RNA type aptamer is 5'-GUGGACUAUACCGCGUAAUGCUGCCUCCAC-3' (SEQ ID NO. 8). The preferred HSA-binding nucleic acid of the present invention is preferably consistent with the type of nucleic acid framework, which is convenient for synthesis and preparation. For example, L-RNA or L-DNA can be selected.

In addition, nucleic acids modified with dendritic alkyl chains can also bind to HSA. For example, J. Am. Chem. Soc. 2017.139.21.7355-7362 reported a DNA cube assembled from dendritic alkyl-modified DNA, which can bind to HSA with high affinity.

For the half-life extension factors of proteins or polypeptides, in order to facilitate their coupling with activated L-nucleic acids, a specific site (such as mutation site, Cys) will be introduced on the half-life extension factor for the coupling of the linker.

For nucleic acid-based half-life extension factors, the sequence can be integrated with the same type of nucleic acid used for assembly and synthesized simultaneously.

### drug library

The present invention provides a drug library comprising: (a) a drug unit; and (b) n half-life extension units;
wherein, the drug unit includes a drug component part and a first nucleic acid component part connected to the drug component part;
the half-life extension unit comprises a half-life extension component part and a second nucleic acid component part connected to the half-life extension component part;
and one of the first nucleic acid component part of the drug unit and the second nucleic acid component part of at least one half-life extension unit can form a base-complementary pairing structure (for example, a double-stranded pairing structure), thereby forming a " drug unit - half-life extension unit" complex;
among them, n is a positive integer ≥ 1.

As used herein, "complementary" includes direct complementary pairing or complementation of the single-stranded complementary sequences of one or more (e.g., 2, 3, 4, 5, 6) auxiliary complementary nucleic acid molecules (i.e., nucleic acid F) to form a base-complementary pairing structure (e.g., double-stranded pairing structure).

In a preferred embodiment of the present invention, the auxiliary complementary nucleic acid molecule (i.e., nucleic acid F) is a multimerization assisting molecule (chain).

In a preferred embodiment of the present invention, the drug unit and the half-life extension unit form a multimer in the presence of the multimerization assisting molecule (chain).

Typically, the "complementary" is a direct complementary pairing.

In a preferred embodiment of the present invention, the "complementary" refers to a base-complementary pairing structure formed by the complementation of the single-stranded complementary sequence of an auxiliary complementary nucleic acid molecule (i.e., nucleic acid F).

In another preferred embodiment of the present invention, the "complementary" refers to the formation of a base-complementary pairing structure through the complementation of the single-stranded complementary sequences of two auxiliary complementary nucleic acid molecules (i.e., nucleic acid F) (as shown in Figure 2 ).

The library of the present invention contains at least one drug unit and at least one half-life extension unit, and the drug unit and the half-life extension unit are assembled to form a drug, and a preferred drug has the structure of Formula I above.

In another preferred embodiment of the present invention, the library of the present invention contains 1 drug unit and at least one (e.g. 2, 3, 4) half-life extension units.

Typically, the drug unit has the structure of Formula II above.

Typically, the half-life extension unit has the structure of Formula III above.

Due to the specific structure of the drug unit and the half-life extension unit of the present invention, as a result, not only can drugs be assembled rapidly, but the resulting drugs have unexpectedly extended half-lives (significantly increased in vivo stability), and can persist and remain active in the body for a long time without being rapidly degraded.

For example, in the treatment of cancer (or other metabolic diseases), multiple targets and multiple pathways are often involved, and each patient not only has different etiologies, different constitutions, different ages, but also has tumor heterogeneity or different subtypes of disease within a patient, often have different pharmacokinetics and metabolic pathways, so that drugs with multiple targets and different half-lives are often needed. With the development of personalized therapy or precision therapy technology, there is an urgent need in the field to develop protein drugs (such as multispecific antibodies) that can be quickly prepared, have low cost, good targeting, good stability, and controllable (adjusted) half-life. The library of the present invention fulfills this need.

It should be understood that although the drug library of the present invention contains or mainly contains or completely contains the above-mentioned drug units and half-life extension units of the present invention, the drug library also contains other therapeutic agents, especially other protein drugs, representative examples include (but are not limited): antibodies, compounds, fusion proteins. For example, a library of the present invention may additionally contain one or more conventional antibodies with therapeutic effect, or conventional drugs with extended drug half-life, or conventional metabolic modulators.

It should be understood that the number of half-life extension units in the library of the present invention is not limited, and can be any positive integer n of ≥2. For example, n is any positive integer from 3-100; n is 3-50; more preferably n is 5-20; most preferably n is 5-8.

In addition, in the present invention, the antibody component in the half-life extension unit is not particularly limited, and representative examples (selected from the group consisting of): single-chain antibody, nanobody, Fab, monoclonal antibody, and a combination thereof.

For the library of the present invention, various sources of proteins and polypeptides can be used to prepare drug units; antibodies from various sources can be used to prepare half-life extension units. An outstanding feature of the drug library of the present invention is that antibody fragments expressed by prokaryotic systems (e.g., E. coli) or eukaryotic systems (e.g., yeast, CHO cells) can be used, thereby greatly reducing production costs.

Typically, in use, according to needs (such as the condition and diagnosis results of a certain individual, the protein drug information required for treating the disease of the object to be treated, including the type of protein drug, the mode of administration, and the pharmacokinetics), select the corresponding drug unit and half-life extension unit (including the type and number), and easily complete the drug assembly through the nucleic acid complementary framework. For example, in application, according to the target situation of the patient's disease, the metabolic situation, correspondingly determine the number or ratio of half-life extension units (for example, 2, 3, 4, or more than 4), and then assemble.

When preparing a drug, the corresponding drug units and half-life extension units that can be directly or indirectly paired and coupled to each other are selected from the library and mixed according to the desired antibody ratio, and the assembly process can be completed within 1 minute.

In the library of the present invention, the nucleic acid components (elements) of the drug unit and the nucleic acid components (elements) of the half-life extension unit can be designed into high-polymer frameworks such as dimers, trimers, and tetramers through sequence design, thereby the preparation of a multimer carrying 3 or even 4 half-life extension units in one drug molecule, which cannot be easily achieved by traditional drug half-life design and preparation techniques, is completed.

Once assembled to form a multimeric protein drug, it can be administered to the respective individual for therapeutic purposes.

### Left-handed nucleic acid (L-Nucleic Acid)

Left-handed nucleic acid refers to the existence of a mirror image relative to the natural right-handed nucleic acid (D-nucleic acid), which can be divided into left-handed DNA (L-DNA) and left-handed RNA (L-RNA). The levogyration (chiral center) is found primarily in the ribodesose or ribose portion of nucleic acids, mirror image flipped. Therefore, L-nucleic acid cannot be degraded by nucleases (e.g., exonuclease, endonuclease) that are ubiquitous in plasma.

### The drug with extended half-life of the present invention

The drug with extended half-life of the present invention refers to a drug with extended half-life formed by a drug unit and n kinds of half-life extension units through nucleic acid complementation to form a base-complementary pairing structure (for example, a double-stranded pairing structure), wherein n is a positive integer ≥ 1; wherein, the drug unit comprises a drug component part and a first nucleic acid component part connected with the drug component part, and the half-life extension unit comprises a half-life extension component part and a second nucleic acid component part connected with the half-life extension component part and the nucleic acid component part of one of the drug units and the nucleic acid component part of at least one half-life extension unit form a base-complementary pairing structure (for example, a double-stranded pairing structure) through complementation.

In another preferred embodiment, the nucleic acid component part is resistant to nuclease degradation.

In another preferred embodiment, the nucleic acid component part is selected from the group consisting of L- nucleic acid, peptide nucleic acid, locked nucleic acid, thio-modified nucleic acid, a 2'-fluoro-modified nucleic acid, a 5-hydroxymethylcytosine nucleic acid, and a combination thereof.

The drug of the present invention can be formed, for example, by assembling a drug unit of Formula II and a half-life extension unit of Formula III.

Typically, a multimeric drug is one composed of a drug unit assembled with multiple half-life extension units, such as di-, tri-, tetra-, penta- or hexamers.

In a preferred example, the medicament of the present invention is linked by left-handed nucleic acid. The research of the present invention shows that nucleic acid is a double-stranded molecule that can rapidly perform specific pairing. Therefore, if the half-life extension component part (such as HSA, single-chain antibody, nanobody, Fab, etc.) is coupled to the nucleic acid single-strand, by coupling the drug component part with the nucleic acid single strand, the nucleic acid sequence can be designed so that two or more nucleic acid single strands can be quickly paired, thereby guiding the rapid assembly of the drug unit with one or more half-life extension units to complete the preparation of drugs with extended half-life.

In the present invention, in order to achieve the effect of prolonging the half-life of a drug, it is necessary to adopt a drug unit with a specific structure and a half-life extension unit. In a preferred example, by using a left-handed nucleic acid (such as L-DNA, L-RNA, etc.) instead of a right-handed nucleic acid (such as D-DNA, D-RNA), the half-life of the drug can be significantly improved and prolonged. One reason is that L-nucleic acid cannot be degraded by exonuclease, endonuclease, etc. existing in the human body, so that the drug mediated by the self-assembly of L-nucleic acid (left-handed nucleic acid) will be extremely stable in vivo.

### Preparation method

### 1. Design and preparation of L-nucleic acid strand framework

According to the present invention, the L-nucleic acid strand framework is formed by two or more L-nucleic acid single strands through base pairing. The 5' or 3' end of each L-nucleic acid single chain is activated to be a group available for subsequent modification (such as NH₂, etc.), and then use one end of a linker (such as SMCC, SM(PEG), SPDP, etc.) to couple with the activating group on the L-nucleic acid single strand. The L-nucleic acid with the linker can be assembled into the desired L-nucleic acid chain framework. After it is determined that the L-nucleic acid with the linker can successfully self-assemble into a framework, the L-nucleic acid single chain with the linker can be coupled to the antibody for subsequent assembly, respectively.

The L-nucleic acid framework of the present invention can basically be prepared by the following steps.

### 1.1 Design of L-nucleic acid single strands for rapid self-assembly

Determine the required number N of half-life enhancing modules (for example, three half-life enhancing modules); determine the required number M of L-nucleic acid single strands according to the number N of half-life enhancing modules (M>=N+1); design a corresponding number of L- Nucleic acid single-stranded sequences, adjust the stability of the target nucleic acid framework by increasing or decreasing the number of base pairings, reducing the possibility of non-specific pairing between nucleic acid strands.

According to a preferred embodiment of the present invention, in order to design a tetrameric L-nucleic acid framework (M=4), four L-nucleic acids that can be paired according to certain rules are designed (as shown in FIG. 2). Wherein, any one L-nucleic acid single strand can be specifically complementary paired with the other two L-nucleic acid single strands, but not paired with the fourth one. And the absolute value of the Gibbs energy change ΔG of the specific complementary pairing is much larger than that of the non-specific pairing. For example, in this preferred embodiment, the absolute value of the Gibbs energy change ΔG for the specific complementary pairing of each arm is about 26 kilocalories per mole (kcal/mole) while the nonspecific pairings are all less than 7 kcal per mole (kcal/mole), which means that the assembly of tetramers is more likely to occur than non-specific pairwise pairing, and the tetramer form is the most stable in the reaction system. Tetrameric L-nucleic acid frameworks can link 1-3 half-life enhancing factors.

### 1.2 Activation of L-DNA or L-RNA

Activation of the L-nucleic acid includes modification of the active group at its 5' or 3' end and subsequent linker conjugation. The active group modification can be customized by a nucleic acid synthesis company; the linker generally has a bifunctional group, that is, one end can be coupled to an active group of nucleic acid, and the other end can be connected to a specific site (such as SH) on the antibody.

According to a preferred embodiment of the present invention, all L-nucleic acids constituting the framework are modified with NH₂ at the 5' end, and then a linker, i.e. a bispecific functional group cross-linking reagent SMCC (4-(N-maleimidomethyl) Cyclohexane-1-carboxylate succinimidyl ester sodium salt) is coupled to NH₂ on nucleic acids via an amido bond. At this time, the maleimide group at the other end of the linker is in a free state, which can be used for subsequent coupling of the sulfydryl (SH) on the antibody, thus completing the activation of the L-nucleic acid.

### 2. Preparation method of antibody-L-nucleic acid complex

First, the 5' or 3' end of L-nucleic acid is modified with NH₂, and then the following main preparation methods can be used depending on the linker, wherein the functional group at one end of the linker is NHS (N-hydroxysuccinimide) or Sulfo- NHS (N-hydroxy succinimide sodium sulfonate), for rapid coupling of the NH₂ group at one end of the L-nucleic acid. The linkers containing bispecific functional groups first react with NH₂ of L-nucleic acid, and secondly, after reducing the sulfhydryl group on the antibody, the other end group reacts with the sulfhydryl group to form a stable chemical bond.

**2.1 Maleimide.** The linker used to couple the sulfhydryl group on the antibody is maleimide. Maleimide can rapidly react with free sulfhydryl groups on antibodies to form thioether bonds. Common linkers are SMCC (4-(N-maleimidomethyl)cyclohexane-1-carboxylate succinimide eater), SM(PEG) (polyethylene glycol modified 4-(N-maleimidomethyl)cyclohexane-1- carboxylate succinimide eater) and the like.

**2.2 Haloacetyl.** The linker used to couple the sulfydryl on the antibody is haloacetyl, such as iodine, bromoacetyl. Halogen ion can be replaced with sulfhydryl groups on antibodies by nucleophilic interaction to form stable thioether bonds. Common linkers are SBAP (N-maleimidomethyl[4-bromoacetyl]aminobenzoate), SIAB (N-maleimidomethyl[4-iodoacetyl]aminobenzene) formate) etc.

**2.3 Pyridyldithiol.** The linker used to couple the sulfydryl on the antibody is thiopyridine. Thiopyridines can react with free sulfhydryl groups to form disulfide bonds. Common linkers are SPDP (3-(2-pyridinedithio)propionic acid N-hydroxysuccinimide eater) and the like.

### pharmaceutical composition

The present invention also provides a composition. In a preferred example, the composition is a pharmaceutical composition, which contains the above-mentioned antibody or its active fragment or its fusion protein, and a pharmaceutically acceptable carrier. Generally, these materials can be formulated in a non-toxic, inert and pharmaceutically acceptable aqueous carrier medium, usually at a pH of about 5-8, preferably at a pH of about 6-8, although pH can vary depending on the nature of the substance being formulated and the condition being treated. The formulated pharmaceutical compositions can be administered by conventional routes including, but not limited to, oral, respiratory, intratumoral, intraperitoneal, intravenous, or topical administration.

The pharmaceutical composition of the present invention can be directly used for treatment (e.g., anti-tumor treatment), and thus can be used to prolong the half-life of the drug. In addition, other therapeutic agents can be used simultaneously.

The pharmaceutical composition of the present invention contains a safe and effective amount (such as 0.001-99wt%, preferably 0.01-90wt%, more preferably 0.1-80wt%) of the above-mentioned monoclonal antibody (or its conjugate) of the present invention and a pharmaceutical an acceptable carrier or excipient. Such carriers include, but are not limited to, saline, buffers, dextrose, water, glycerol, ethanol, and combinations thereof. The drug formulation should match the mode of administration. The pharmaceutical composition of the present invention can be prepared in the form of injection, for example, prepared by conventional methods with physiological saline or an aqueous solution containing glucose and other adjuvants. Pharmaceutical compositions such as injections and solutions are preferably manufactured under sterile conditions. The active ingredient is administered in a therapeutically effective amount, e.g., about 1 microgram/kg body weight to about 10 mg/kg body weight per day. In addition, the polypeptides of the present invention may also be used with other therapeutic agents.

When the pharmaceutical composition is used, a safe and effective amount of the immunoconjugate is administered to the mammal, wherein the safe and effective amount is generally at least about 10 micrograms/kg body weight, and in most cases no more than about 8 mg/kg body weight, preferably the dose is about 10 micrograms/kg body weight to about 1 mg/kg body weight. Of course, the specific dosage should also take into account the route of administration, the patient's health and other factors, which are all within the skill of the skilled physician.

### The main advantages of the present invention are:

(1) The half-life extension module of the present invention can be mediated by L-nucleic acid chain to complete the assembly with the target protein within one minute;
(2) The half-life extension factor in the half-life enhancing module of the present invention has a wide range of modification space, and HSA or any form of HSA-binding protein, small molecule and polypeptide (such as anti-HSA single-chain antibody, nanobody, Fab) can be selected;
(3) The half-life enhancing module of the present invention can be stored separately for future use, and then coupled with various protein or polypeptide drugs through simple nucleic acid pairing assembly;
(4) The half-life extension module of the present invention can flexibly prepare long-half-life medicines containing different numbers of half-life extension modules as required, so as to flexibly adjust the pharmacokinetic properties;
(5) In the half-life extension module of the present invention, the half-life extension factors are all low-cost, easy-to-prepare proteins or polypeptides, such as anti-HSA nanobodies that can be expressed in Escherichia coli, HSA protein with a concentration of up to 50g/L in human blood, etc. Therefore, the long-acting drugs prepared based on this module have the advantages of simple preparation, strong versatility and low cost;
(6) The present invention provides a flexible preparation method for linking a drug to an FcRn targeting factor, and an application of prolonging the half-life of the drug in animals through the FcRn-mediated drug recovery pathway.

The present invention will be further described below in conjunction with specific embodiments. It should be understood that these examples are only used to illustrate the present invention and not to limit the scope of the present invention. The experimental method of unreceipted specific conditions in the following examples, usually according to normal conditions, such as people such as Sambrook, molecular cloning: conditions described in laboratory manual (New York: Cold Spring Harbor Laboratory Press, 1989), or according to manufacture conditions recommended by the manufacturer. Percentages and parts are by weight unless otherwise indicated. The experimental materials involved in the present invention can be obtained from commercial sources unless otherwise specified.

### Example 1 Design of tetrameric DNA structural framework

Four L-nucleic acids were designed that could be paired in a quadrilateral shape (as shown in Figure 2). Wherein, any one L-nucleic acid single strand can be specifically complementary paired with the other two L-nucleic acid single strands, but not paired with the fourth one. And the absolute value of the Gibbs energy change ΔG of the specific complementary pairing is much larger than that of the non-specific pairing, and the absolute value of the Gibbs energy change ΔG of the specific complementary pairing of each arm is greater than 25 kcal per mole (kcal/mole), and non-specific pairings are all less than 7 kcal/mole (kcal/mole), which means that tetramer assembly is more likely to occur than non-specific pairwise pairing. The tetrameric form is the most stable in the reaction system.

The four L-DNA single-stranded sequences designed according to the above principles are as follows (from 5' to 3'):
Strand 1 (L-DNA1): SEQ ID NO. 1
   5'-AAGAGGACGCAATCCTGAGCACGAGGTCT-3'
Strand 2 (L-DNA2): SEQ ID NO. 2
   5'-AACTGCTGCCATAGTGGATTGCGTCCTCT-3'
Strand 3 (L-DNA3): SEQ ID NO.3
   5'-AATGAGTGCATTCGGACTATGGCAGCAGT-3'
Strand 4 (L-DNA4): SEQ ID NO. 4
   5'-AAGACCTCGTGCTCACCGAATGCACTCAT-3'

The 5' end is modified with an NH₂ group for coupling to the NHS of SMCC. The base sequence of any one strand is complementary to the other two strands, and the paired Gibbs energy change ΔG for each fraction is about -34 kilocalories per mole (kcal/mole).

### Example 2 Synthesis and Verification of Tetrameric DNA Framework

The NH₂-modified L-DNA single strands at the 5' end were synthesized by biotechnology service companies (e.g., Chemgene Inc., Biosyn Inc., etc.). The sequences of the four single strands were shown in Example 1.

L-DNA single strands were dissolved in phosphate buffer (50 mM NaH₂PO₄, 150 mM NaCl, pH 7.0) to prepare a stock solution with a final concentration of 200 uM. Dissolve the SMCC powder with dimethyl sulfoxide (DMSO) to make a fresh 250 mM SMCC stock solution. Add 10-50 times molar amount of SMCC stock solution to L-DNA single-strand stock solution, mix quickly and react at room temperature for 30min-2h. After the reaction was completed, 10% volume of 1M Tris-HCl (pH 7.0) was added to the reaction solution, mixed and incubated at room temperature for 20 minutes to stop excess SMCC and continue the reaction. After the incubation, add 2 times the volume of 100% absolute ethanol of the reaction solution, mixed evenly, and placed in a -20 degree refrigerator for 25 minutes to fully precipitate L-DNA. Centrifuged (12000rpm, 10min) to take the precipitate, washed with 1mL of 70% ethanol, remove the supernatant by centrifugation at 12000rpm for lmin, and repeat the washing 5 times to fully remove excess SMCC. The remaining white precipitate was naturally dried in the air for 5-10 min, and then resuspended and dissolved in phosphate buffer to obtain SMCC-L-DNA complex (i.e., SMCC-L-DNA single strand).

The concentration of each SMCC-L-DNA single strand was determined. Take an appropriate amount of four SMCC-L-DNA single strands to be reacted and preheated at 40 degrees for 5 minutes, then mix the four SMCC-L-DNA single strands in equimolar amounts at 40 degrees, and incubated for 1 minute. Take 0.25 ul of SMCC-L-DNA single strand and the reaction product to analyze by 3% agarose gel electrophoresis.

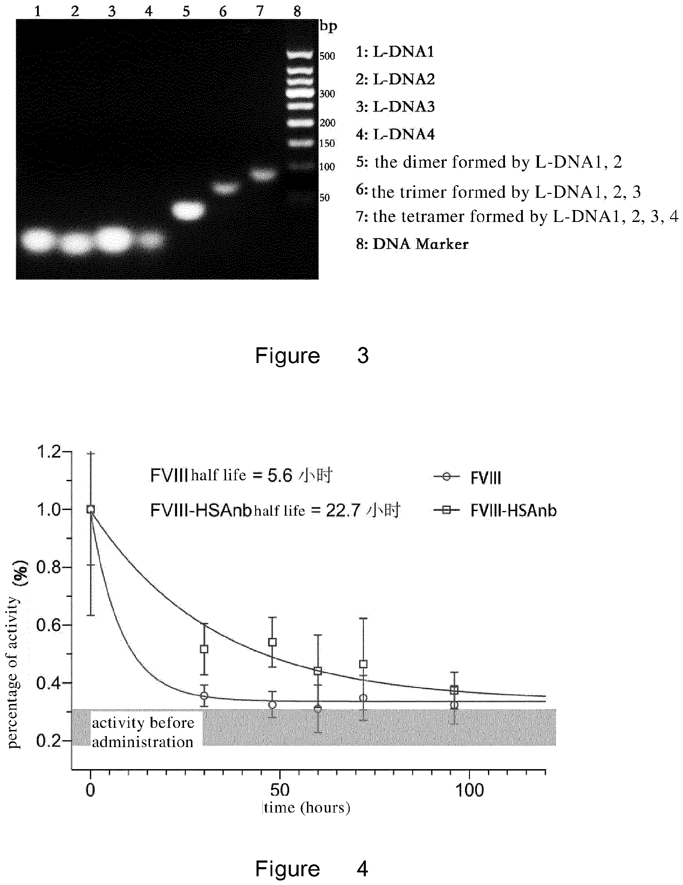

The results are shown in Figure 3. The size of the SMCC-L-DNA single strand is about 25 bp, while the main band formed after mixing is about 100 bp, indicating that four distinct SMCC-L-DNA single strands form a tetrameric framework.

### Example 3 Preparation of half-life extension factor-anti-HSA nanobody mutants

Cysteine mutations were introduced at the carboxy terminus of the anti-HSA Nanobody. Disulfide bonds exist in Nanobodies, but due to their small molecular weight and unusually stable structure, they can be expressed in the cytoplasm of E. coli.

The gene sequence of the anti-HSA Nanobody was optimized to E. coli-preferred codons and then subcloned into the pET21b plasmid.

The amino acid sequence of the anti-HSA Nanobody is SEQ ID NO.5. To facilitate purification, double Strep tags were added to the N-terminus of Nanobodies.
SEQ ID NO.5, amino acid sequence of anti-HSA Nanobody mutant:

SAWSHPQFEKGGGSGGGSGGSAWSHPQFEKENLYFQSEVQLVESGGGL
VQPGNSLRLSCAASGFTFSSFGMSWVRQAPGKGLEWVSSISGSGSDTLYADS
VKGRFTISRDNAKTTLYLQMNSLRPEDTAVYYCTIGGSLSRSSQGTLVTVSSG
SC

Take 1 ul of the constructed expression vector to transform E. coli BL21 (DE3) respectively, pick a single colony of transformed BL21 (DE3) into LB medium (containing 100 ug/mL ampicillin), and cultivate at 37 degrees to OD600=0.7, Expression was induced by adding IPTG at a final concentration of 1 mM and cultured at 37°C for 3 to 4 hours. After the expression was completed, the cells were collected by centrifugation, resuspended in phosphate buffer (50 mM NaH₂PO₄, 150 mM NaCl, pH 7.0), protease inhibitor cocktail (Sigma) (a SIGMAFAST^{™} protease inhibitor tablet was added to the solution to fully dissolved), disrupted by sonication. Add DNaseI hydrolase and incubated on ice for 1 hour. After the incubation, the bacterial solution was centrifuged at 17,000 rpm for 20 minutes to collect the supernatant. Nanobodies in the supernatant were purified with a Strep affinity column. After the supernatant was passed through the column at a speed of 0.25 ml/min, wash the column with a large volume of Tris buffer (50 mM Tris-HCl, 150 mM NaCl, pH 7.4) at a flow rate of 1 ml/min until the impurity protein no longer flows out (according to UV absorption on an AKTA protein chromatography system), nanobodies bound to the column were gradient eluted with a Strep-Tactin elution buffer (50 mM Tris-HCl, 150 mM NaCl, 2.5 mM Dethiobiotin pH 7.4).

Results: High-purity anti-HSA nanobody mutants are obtained.

### Example 4 Conjugation and purification of Nanobody-L-DNA

The purified single-chain antibody was incubated with a 10-50-fold molar ratio excess of a reducing agent (such as TCEP, DTT, mercaptoethanol, etc.) at room temperature for 30 min. After the incubation, PD-10 desalting column was used to quickly remove the reducing agent in the reaction system, and the buffer was replaced with phosphate buffer (50 mM NaH₂PO₄, 150 mM NaCl, pH 7.0). After measuring the concentration of single-chain antibody, immediately add SMCC-L-DNA single-chain (prepared in Example 2) in excess of 1-4 times molar ratio, mixed well, and reacted at room temperature for 1 hour.

Unreacted and excess SMCC-L-DNA single strands were removed using a Strep affinity column, and Nanobodies and Nanobody-L-DNA mixtures were collected. Replace its buffer with the loading buffer of the anion exchange column.

Since nucleic acids such as DNA are negatively charged, the Nanobody-L-DNA was further separated and purified using an anion exchange column (HiTrap Q HP column) to remove unreacted Nanobody. The separation process was achieved by gradient elution, the loading buffer was 50 mM Tris-HCl, pH 8.5, and elution buffer was 50 mM Tris-HCl, 1 M NaCl pH 8.5, 0-100% elution buffer for gradient elution, unreacted Nanobody and Nanobody-L-DNA peaks successively (as shown in Figure 5, lane D10). Nanobody-L-DNA was collected, concentrated and buffer exchanged with a PD-10 desalting column to 50 mM NaH₂PO₄, 150 mM NaCl, pH 7.4.

### Example 5 Self-assembly of half-life enhancing module and target protein drug

The following takes the design of a long-acting factor eight (FVIII) drug for the treatment of hemophilia A as an example to describe the self-assembly process of the half-life enhancing module and the target protein drug. In this embodiment, the eight-factor is used as the target protein drug, and the eight-factor may be a blood-derived eight-factor extracted from human blood, or a recombinant eight-factor obtained by genetic engineering.

The eight-factor was mixed with a 10-50-fold molar ratio excess of a reducing agent (such as TCEP, DTT, mercaptoethanol, etc.) and incubated at room temperature for 30 min. After the incubation, PD-10 desalting column was used to quickly remove the reducing agent in the reaction system, and the buffer was replaced with phosphate buffer (50 mM NaH₂PO₄, 150 mM NaCl, pH 7.0). After determining the concentration of the eight-factor, immediately add SMCC-L-DNA single strands (prepared in Example 2) in excess of 1-4 times molar ratio, mixed well, and reacted at room temperature for 1 hour. The product after the reaction is the drug unit: eight-factor-L-DNA. As shown in Figure 6a, the reacted drug unit band shifted upward.

The concentrations of anti-HSA Nanobody-L-DNA and eight-factor-L-DNA were determined respectively. Take an appropriate amount of the above components and preheated at 40 degrees for 5 minutes, then mix the HSA Nanobody-L-DNA and eight-factor-L-DNA at a 1:1 molar ratio at 40 degrees, and incubated for 1 minute. So far, the FVIII-HSAnb containing the half-life enhancing module and the active part of the eight-factor has been assembled, that is, the long-acting eight-factor. The schematic diagram of the assembled structure of FVIII-HSAnb is shown in Fig. 6b. As shown in Figure 6a, compared with the reacted drug unit (eight-factor-L-DNA), the long-acting eight-factor band shifted upward.

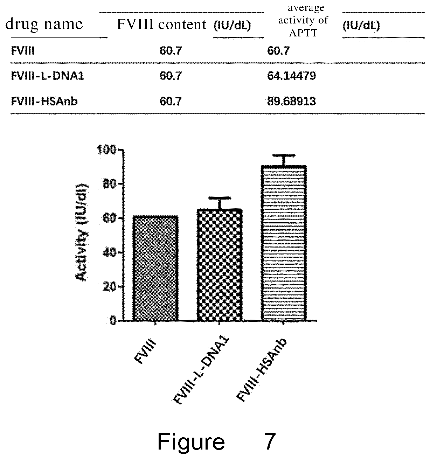

The activity of samples containing the same amount of FVIII protein was determined by APTT kit (Sun Biotechnology). The results are shown in Figure 7. The activity of FVIII-L-DNA1 is similar to that of the unreacted drug unit, but the activity of the long-acting eight-factor is significantly increased (-50%).

The half-life prolongation effect of FVIII-HSAnb in vivo was verified using FVIII-deficient mice aged 6-8 weeks. Eight mice were randomly divided into experimental group and control group, and FVIII-HSAnb and FVIII were intravenously injected with 5 IU/mice respectively. Blood sampling time was the day before administration, 5 min, 30 h, 48 h, 60 h, 72 h and 96 h after administration. After blood collection, the plasma was quickly separated and stored in a -80°C freezer. After all plasma samples were obtained, FVIII activity was detected using the APTT method.

According to calculations, the half-lives of FVIII and FVIII-HSAnb were 5.6 h and 22.7 h, respectively (as shown in Figure 4).

Therefore, the FVIII-HSAnb designed by the present invention can prolong the half-life by about 4 times compared with ordinary FVIII.

### Discussion

IgG-type monoclonal antibodies (monoclonal antibodies) have the longest half-life among immunoglobulin antibodies, up to 21 days. Therefore, IgG-type antibodies are currently the main type of therapeutic antibodies.

The long half-life and high serum concentration of IgG-type mAbs are achieved by binding to the neonatal receptor (FcRn) and entering into its mediated metabolic regulation. The Fc portion of IgG can specifically bind to FcRn in a pH-dependent manner. When the IgG monoclonal antibody is endocytosed by hematopoietic cells or vascular endothelial cells in the blood through pinocytosis, it can enter the endosome and bind to FcRn therein, thereby entering the recycling pathway and avoiding entry into the degradation pathway and degraded by the lysosome, thereby recycling to the cell surface, and dissociating from FcRn under neutral physiological conditions (pH 7.4), releasing it into the blood.

Albumin is another protein that can prolong its half-life by binding to FcRn, and its half-life can be as long as 19 days. Human serum albumin can be divided into four domains I-IV, of which domain III is the part that binds to FcRn, and domain I contains a free cysteine residue (Cysteine 34, Cys 34).

For protein or polypeptide drugs with long half-life requirements, such as cytokine drugs, coagulation factors, etc., FcRn-binding proteins such as Fc and HSA are better choices.

The half-life extension method of Fc or HSA fusion proteins will generate new epitopes at the fusion site of the target protein or polypeptide with Fc/HSA. In addition, Fc can only be fused to the C-terminus, while HSA can only be fused to the N-terminus or C-terminus of the target protein or polypeptide. Furthermore, this method is not applicable to native proteins or peptides.

The present invention develops for the first time a chemical coupling method, which uses chemical molecules or linkers to specifically achieve heterologous coupling of proteins or polypeptides with FcRn binding modules (HSA or HSA binding modules, etc.), thereby prolonging the protein drug half-life, enhancing their pharmacokinetic properties.

Compared with Fc or HSA fusion protein, the method of the present invention is a more flexible solution, which can carry out site-directed or non-site-directed modification of various groups (such as SH, NH₂, active groups on unnatural amino acids, etc.) of the target protein or polypeptide, and can also be applied to the modification of natural proteins or polypeptides.

Furthermore, the methods of the present invention do not or substantially do not introduce new epitopes.

All the documents cited herein are incorporated into the invention as reference, as if each of them is individually incorporated. Further, it would be appreciated that, in light of the above described teaching of the invention, the skilled in the art could make various changes or modifications to the invention, and these equivalents would still be in the scope of the invention defined by the appended claims of the application.

## Claims

1. A drug library comprising: (a) a drug unit; and (b) n half-life extension units;
wherein, the drug unit comprises a drug component part and a first nucleic acid component part connected to the drug component part;
the half-life extension unit comprises a half-life extension component part and a second nucleic acid component part connected to the half-life extension component part;
and one of the first nucleic acid component part of the drug unit and the second nucleic acid component part of at least one half-life extension unit can form a base-complementary pairing structure to form a "drug unit-half-life extension unit" complex;
wherein, n is a positive integer ≥ 1.

2. The drug library of claim 1, wherein the "drug unit-half-life extension unit" complex has a structure as shown in Formula I:
To-[Lo-Ao]n (I)
wherein
T₀ is a drug component part;
L₀ is a nucleic acid linker (linker), the nucleic acid linker is used to connect T₀ and A₀ and contains a pairing structure based on the base complementary of the first nucleic acid component part and the second nucleic acid component part;
A₀ is an FcRn binding component, or a binding component that binds to an FcRn binding protein such as serum albumin;
n is a positive integer ≥1.

3. The drug library of claim 1, wherein the drug unit has a structure shown in Formula II:
T-X1-L1-Y1-W1-Z1 (II)
the half-life extension unit has a structure shown in Formula III:
A-X2-L2-Y2-W2-Z2 (III)
wherein
T is a drug component part;
A is a half-life extension component part;
X1 and X2 is each independently none or a redundant peptide;
L1 and L2 is each independently a linker molecule;
Y1, Y2 and Z1, Z2 is each independently none or a redundant nucleic acid;
W1 and W2 is each independently a nucleic acid sequence selected from the group consisting of L-nucleic acid, peptide nucleic acid, locked nucleic acid, thio-modified nucleic acid, a 2'-fluoro-modified nucleic acid, a 5-hydroxymethylcytosine nucleic acid, and a combination thereof;
"-" is a covalent bond;
wherein the nucleic acid W1 of the drug unit has at least one complementary pairing region, and the nucleic acid W2 of the half-life extension unit has at least one complementary pairing region, and the two are partially or completely complementary.

4. The drug library of claim 3, wherein the drug component part is selected from the group consisting of an antibody, an activation receptor or inhibition receptor, a ligand of the protein, a biologically active enzyme, a nucleic acid drug, a small molecule drug, and a combination thereof.

5. The drug library of claim 3, wherein the half-life extension component part is selected from the group consisting of: a natural albumin (Albumin), recombinant albumin, anti-albumin antibody (including nanobody, single chain antibody, Fab, monoclonal antibody), a nucleic acid aptamer (aptamer) that specifically bind to albumin, a protein and a nucleic acid aptamer (aptamer) that binds directly to FcRn, any protein with a long half-life, and a combination thereof.

6. The drug library of claim 3, wherein the linker molecule L1 and L2 each independently has a bifunctional linker, which can be coupled with the modified ends with modifying groups of nucleic acids W1, W2 or Y1, Y2 and T or A or the specific linking site of XI, X2.

7. A method assembling a drug with extended half-life, such as a protein drug, comprising:
(a) selecting a drug unit and a half-life extension unit from the drug library of claim 1 based on pharmaceutical information; and
(b) mixing the drug unit and at least one half-life extension unit to assemble a drug with extended half-life.

8. A drug with extended half-life, the drug with extended half-life is a drug with extended half-life formed by a drug unit and n kinds of half-life extension units through nucleic acid complementation to form a base-complementary pairing structure (for example, a double-stranded pairing structure), wherein n is a positive integer ≥ 1;
wherein, the drug unit comprises a drug component part and a first nucleic acid component part connected to the drug component part, and the half-life extension unit comprises a half-life extension component part and a second nucleic acid component part connected to the half-life extension component part, and the nucleic acid component part of one of the drug units and the nucleic acid component part of at least one half-life extension unit form base-complementary pairing structures (e.g., double-stranded pairing structures) by complementation.

9. The drug with extended half-life of claim 8, wherein the nucleic acid component part is selected from the group consisting of L-nucleic acid, peptide nucleic acid, locked nucleic acid, thio-modified nucleic acid, a 2'-fluoro-modified nucleic acid, a 5-hydroxymethylcytosine nucleic acid, and a combination thereof.

10. A pharmaceutical composition, the pharmaceutical composition comprises
(i) the drug with extended half-life of claim 8 is used as an active ingredient; and
(ii) a pharmaceutically acceptable carrier.
